# FASCICULE DE BREVET EUROPEEN

(11) **EP 1 708 716 B1**
(45) Date de publication et mention de la délivrance du brevet: **01.08.2007**
(21) Numéro de dépôt: 05717490.6
(22) Date de dépôt: 26.01.2005
(51) Int. Cl.: A61K 31/522, A61K 31/201, A61P 3/00

(54) **COMPOSITION AMAIGRISSANTE PAR VOIE ORALE COMPRENANT DE L'ACIDE LINOLEIQUE CONJUGUE ET DE LA CAFEINE**
ORALE DIÄTZUSAMMENSETZUNG, ENTHALTEND KONJUGIERTE LINOLSÄURE UND COFFEIN
ORAL DIETING COMPOSITION COMPRISING CONJUGATED LINOLEIC ACID AND CAFFEINE

(30) Priorité: 27.01.2004 FR 0400739
(43) Date de publication de la demande: 11.10.2006
(73) Titulaire: Institut Phytoceutic, 83600 Frejus (FR)
(72) Inventeur: VERNEAU, Bernadette, F-83600 Frejus (FR)
(74) Mandataire: Ahner, Francis
(86) Numéro de dépôt international: PCT/FR2005/000166
(87) Numéro de publication internationale: WO 2005/082371

(56) Documents cités:
- WO-A-01/17498
- WO-A-01/60331
- US-B1- 6 413 545

## Description

La présente invention concerne une composition amaigrissante pour une administration par voie orale comprenant une association d'acide linoléique conjugué et de caféine à titre de principe actif.

L'acide linoléique conjugué (CLA) est un mélange d'isomères de l'acide linoléique qui sont naturellement présents dans le lait et les produits laitiers, ainsi que dans la viande des ruminants. Le terme CLA inclut l'ensemble des isomères de configuration et de position de l'acide octadécadiénoïque (C18 :2), en particulier la forme cis et trans des acides 9,11-, 10,12- et 11,13- octadécadiénoïques.

Il a été démontré que l'administration orale de CLA permet de réduire la masse grasse (graisses stockées) au profit de la masse maigre (muscles), aussi bien chez l'homme que chez les animaux de boucherie. La dose efficace est de 1 à 2 g/jour.

Deux mécanismes expliqueraient cette activité intéressante. D'une part, le CLA modifie le métabolisme des lipides et en particulier réduit leur stockage sous forme de graisses. D'autre part, le CLA augmente la dépense énergétique de l'organisme (ou thermogenèse) et favorise ainsi l'utilisation de graisses de réserve comme source d'énergie.

La caféine est un alcaloïde présent dans de nombreux végétaux, en particulier dans les grains du caféier, les feuilles du thé ou les graines du kolatier. Outre son activité stimulante psychique et diurétique, la caféine est aussi connue comme agent stimulant la thermogenèse et donc comme agent amaigrissant par voie orale.

Or la demanderesse a découvert de manière tout à fait inattendue qu'une association de CLA et de caféine administrée par voie orale permet de stimuler encore plus ces mécanismes d'élimination des graisses stockées qui conduisent à la perte de poids. Il a été démontré que le CLA et la caféine agissent en synergie.

La présente invention a pour objet une composition amaigrissante pour une administration par voie orale comprenant :
- une association d'acide linoléique conjugué et de caféine à titre de principe actif, et
- un support acceptable pour une administration par voie orale.

L'invention concerne plus particulièrement une composition amaigrissante pour une administration par voie orale comprenant:
- une association d'acide linoléique conjugué et de caféine à titre de principe actif avec un ratio massique acide linoléique conjugué/caféine compris entre 1 et 15, de préférence entre 1 et 6, et
- un support acceptable pour une administration par voie orale.

Le support acceptable pour une administration par voie orale peut être toute substance physiologiquement acceptable, liquide, solide ou pâteuse, plus ou moins inerte, à laquelle on incorpore ledit principe actif afin d'en faciliter la préparation ainsi que l'administration orale et d'en conditionner la consistance, la forme ainsi que le volume.

Ce support peut être en particulier un support alimentaire. Ainsi, ladite composition peut avantageusement se présenter sous la forme de café soluble, c'est-à-dire du café atomisé ou lyophilisé, en poudre, soluble dans l'eau, obtenu exclusivement à partir du café torréfié par des méthodes physiques utilisant l'eau comme seul agent d'entraînement ne provenant pas du café.

En effet, la source la plus commune en caféine est le café que l'on consomme en général sous forme de boisson. La teneur en caféine peut varier grandement suivant le type de café ou le mode de préparation. Le tableau qui suit donne tout de même un ordre de grandeur approximatif pour une tasse de 237 ml. Ce tableau permet aussi de constater que la caféine peut provenir d'autres boissons comme le thé ou de boissons gazeuses à base de cola, mais que leur teneur en caféine est inférieure à celle du café.

| Type de boisson | Teneur en caféine |
|---|---|
| Café filtre | 179 mg |
| Café infusé | 135 mg |
| Café percolateur | 118 mg |
| Café instantané | de 75 mg à 106 mg |
| Thé | 30 à 50 mg |
| Boisson gazeuse de type cola | 36 à 50 mg |

La caféine permet de stimuler la thermogenèse, mais seulement à des doses élevées, en particulier à des doses supérieures à 600 mg/jour. Or, à forte dose, la caféine peut provoquer des effets indésirables tels que l'insomnie, l'irritabilité, la tachycardie ou l'hypertension, ou même être toxique. Si l'on associe le CLA à la caféine, alors on diminue la dose journalière nécessaire pour obtenir un effet amaigrissant ou pour une même dose journalière, on augmente l'effet amaigrissant du café.

Toutefois, le CLA se présente sous forme d'une huile qui se disperse très difficilement dans l'eau. Si on ajoute le CLA dans du café liquide, des gouttes d'huile se forment à la surface du liquide, ce qui n'est pas satisfaisant pour le consommateur. Pour réaliser une formulation de café additionnée d'une grande quantité CLA et qui permet ensuite la dispersion parfaite du CLA dans le café liquide reconstitué, on peut associer le CLA à de la lécithine et de la silice colloïdale. Ces deux adjuvants permettent en effet de préparer le CLA sous la forme d'une poudre qui se disperse parfaitement en milieu aqueux.

Ladite composition peut donc se présenter sous forme de poudre, de comprimés, de gélules, de capsules ou de sachets en poudre.

Ladite composition contient avantageusement de la lécithine et de la silice colloïdale pour permettre la bonne dispersion du CLA en milieu aqueux.

Ladite composition peut aussi contenir d'autres principes actifs qui aident à limiter le taux de glucides dans le sang, tels qu'un extrait de café vert contenant de préférence de 5 à 10 % en poids de caféine, et/ou du chlorure de chrome.

Ladite composition peut être utilisée en tant que complément alimentaire, composition diététique ou composition cosmétique, pour en particulier augmenter la perte de poids, dans le cadre d'un traitement esthétique visant à améliorer la silhouette d'une personne.

Ladite composition peut aussi être utilisée comme médicament, notamment destiné à traiter ou prévenir l'obésité. L'objectif thérapeutique en matière d'obésité est défini de la façon suivante : il s'agit soit de permettre au sujet de perdre du poids de façon significative, soit d'aider le sujet à conserver un niveau de poids aussi bas que possible.

La dose efficace pour augmenter la perte de poids est d'au moins 60 mg de caféine et 300 mg de CLA, deux fois par jour.

### Exemple de formulation (1 sachet) :

| | |
|---|---|
| café atomisé (contenant 60 mg de caféine) : | 2390 mg |
| acide linoléique conjugué : | 310 mg |
| lécithine : | 30 mg |
| extrait de café vert (contenant 5 % en poids de caféine) : | 240 mg |
| chlorure de chrome (contenant 12,5 µg de chrome) : | 0,064 mg |
| silice colloïdale : | 30 mg |

Huit volontaires ont pris par voie orale cette formulation à raison de 2 sachets/jour pendant un mois, sans changer leurs habitudes alimentaires, et ont perdu chacun en moyenne plus de 4 kg.

Une étude complémentaire a été réalisée sur 24 personnes présentant un excès de poids, à qui on a administré par voie orale les formulations du tableau suivant, à raison de deux sachets/jour pendant un mois, sans changer leurs habitudes alimentaires.

| **Formulation testée (1 sachet)** | | **Perte de poids observée (en % pondéral)** |
|---|---|---|
| Café atomisé (contenant 60 mg de caféine) | 2390 mg | - 0,6 (N.S.) |
| Acide linoléique conjugué | 310 mg | -1,8 (N.S.) |
| Café atomisé (contenant 60 mg de caféine) | 2390 mg | |
| Acide linoléique conjugué | 310 mg | - 4,2 (p < 0,05) |
| Café atomisé (contenant 60 mg de caféine) | 2390 mg | |
| Acide linoléique conjugué | 310 mg | |
| Extrait de café vert | 240 mg | - 4,8 (p < 0,01) |
| Chlorure de chrome (contenant 12,5 µg de chrome) | 0,064 mg | |

La perte de poids observée pour le café seul ou le CLA seul n'est pas significative. Par contre, elle est augmentée de façon significative lorsque le CLA et la caféine sont associés dans la même formulation.

Ces résultats confirment la synergie entre la caféine et l'acide linoléique conjugué sur la perte de poids.

## Revendications

1. Composition amaigrissante pour une administration par voie orale comprenant :
- une association d'acide linoléique conjugué et de caféine à titre de principe actif, et
- un support acceptable pour une administration par voie orale,
dans laquelle le ratio massique acide linoléique conjugué / caféine est compris entre 1 et 15.

2. Composition selon la revendication 1, **caractérisée en ce que** le ratio massique acide linoléique conjugué/caféine est compris entre 1 et 6.

3. Composition selon les revendications 1 ou 2, **caractérisée en ce qu'**elle contient en outre de la lécithine et de la silice colloïdale.

4. Composition selon l'une quelconque des revendications 1 à 3, **caractérisée en ce qu'**elle contient en outre un extrait de café vert, et/ou du chlorure de chrome.

5. Composition selon l'une quelconque des revendications 1 à 4, **caractérisée en ce qu'**elle se présente sous forme de poudre ou sous forme liquide.

6. Composition selon la revendication 5, **caractérisée en ce qu'**elle se présente sous forme de café soluble.

7. Composition selon l'une quelconque des revendications 1 à 4, **caractérisée en ce qu'**elle se présente sous forme de comprimés, de gélules, de capsules ou de sachets en poudre.

## Claims

1. Slimming composition for administration by the oral route, comprising:
- a combination of conjugated linoleic acid and caffeine as active ingredient, and
- an acceptable carrier for administration by the oral route,
in which the conjugated linoleic acid/caffeine mass ratio is between 1 and 15.

2. Composition according to Claim 1, **characterized in that** the conjugated linoleic acid/caffeine mass ratio is between 1 and 6.

3. Composition according to Claim 1 or 2, **characterized in that** it additionally contains lecithin and colloidal silica.

4. Composition according to any one of Claims 1 to 3, **characterized in that** it additionally contains a green coffee extract, and/or chromium chloride.

5. Composition according to any one of Claims 1 to 4, **characterized in that** it is provided in powdered form or in liquid form.

6. Composition according to Claim 5, **characterized in that** it is provided in the form of soluble coffee.

7. Composition according to any one of Claims 1 to 4, **characterized in that** it is provided in the form of tablets, gelatin capsules, capsules or sachets of powder.

## Patentansprüche

1. Schlanker machende Zusammensetzung für eine Verabreichung auf oralem Wege, umfassend:
- eine Kombination von konjugierter Linolsäure und von Koffein als Wirkstoff und
- einen Träger, welcher für eine Verabreichung auf oralem Wege annehmbar ist,
in welcher das Massenverhältnis von konjugierter Linolsäure zu Koffein zwischen 1 und 15 eingeschlossen liegt.

2. Zusammensetzung nach Anspruch 1, **dadurch gekennzeichnet, dass** das Massenverhältnis von konjugierter Linolsäure zu Koffein zwischen 1 und 6 eingeschlossen liegt.

3. Zusammensetzung nach den Ansprüchen 1 oder 2, **dadurch gekennzeichnet, dass** sie außerdem Lecithin und kolloidales Siliciumdioxid enthält.

4. Zusammensetzung nach einem der Ansprüche 1 bis 3, **dadurch gekennzeichnet, dass** sie außerdem einen Extrakt von grünem Kaffee und/oder Chromchlorid enthält.

5. Zusammensetzung nach einem der Ansprüche 1 bis 4, **dadurch gekennzeichnet, dass** sie in Pulverform oder in flüssiger Form vorliegt.

6. Zusammensetzung nach Anspruch 5, **dadurch gekennzeichnet, dass** sie in Form von löslichem Kaffee vorliegt.

7. Zusammensetzung nach einem der Ansprüche 1 bis 4, **dadurch gekennzeichnet, dass** sie in Form von Tabletten, Hartkapseln, Weichkapseln oder von Pulver enthaltenden Beuteln vorliegt.
